# EUROPÄISCHE PATENTANMELDUNG

(11) **EP 3 176 146 A1**
(43) Veröffentlichungstag der Anmeldung: **07.06.2017**
(21) Anmeldenummer: 15197689.1
(22) Anmeldetag: 03.12.2015
(51) Int. Cl.: C07C 2/82, C07C 7/09

(54) **VERFAHREN UND ANLAGE ZUR HERSTELLUNG EINES KOHLENWASSERSTOFFPRODUKTS**

(71) Anmelder: Linde Aktiengesellschaft, 80331 München (DE)
(72) Erfinder: Schölch, Michael, 82319 Starnberg (DE); Kamann, Martin, 82041 Oberhacing (DE)
(74) Vertreter: m patent group

(57) **Zusammenfassung**

Ein Verfahren (100, 200) zur Gewinnung eines Kohlenwasserstoffprodukts, bei dem ein Methan enthaltendes Gasgemisch einer oxidativen Methankopplung (10) unterworfen wird, und bei dem unter Einsatz der oxidativen Methankopplung ein gasförmiges Stoffgemisch erzeugt wird, das einem oder mehreren Verdichtungsschritten (20) und anschließend einer thermischen Trennung (30) unterworfen wird, wobei in der thermischen Trennung (30) ein Kältemittel eingesetzt wird, das in einem oder mehreren Verdichtungsschritten (40) verdichtet wird, und wobei unter Verwendung des Stoffgemischs eine oder mehrere Fraktionen gebildet werden, die optional in einem oder mehreren weiteren Verdichtungsschritten verdichtet werden, wird vorgeschlagen. Für einen oder mehrere der Verdichtungsschritte (20, 30) werden dabei ein oder mehrere Verdichter (50) eingesetzt, die mittels einer oder mehrerer Gasturbinen (60) angetrieben werden. Eine entsprechende Anlage ist ebenfalls Gegenstand der vorliegenden Erfindung.

## Beschreibung

Die Erfindung betrifft ein Verfahren und eine Anlage zur Gewinnung eines Kohlenwasserstoffprodukts durch oxidative Kopplung von Methan gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben. Beim Dampfspalten werden Kohlenwasserstoffe in Gegenwart von Dampf typischerweise bei Temperaturen von 800 bis 860 °C bei Verweilzeiten von ca. 0,1 bis 0,8 Sekunden gespalten.

Dampfspaltverfahren werden im kommerziellen Maßstab nahezu ausschließlich in beheizten Rohrreaktoren durchgeführt. Durch Dampfspaltverfahren werden dabei, wie allgemein bekannt, Stoffgemische gewonnen, die neben den eigentlichen Zielverbindungen Nebenprodukte und nicht umgesetzte Kohlenwasserstoffe enthalten. Insbesondere werden in Dampfspaltverfahren typischerweise größere Mengen an Methan und Wasserstoff gebildet. Insbesondere diese Komponenten der sogenannten Heizgasfraktion, aber auch beispielsweise Ethan, Ethylen, Propan und Propylen werden herkömmlicherweise durch thermische Trennschritte voneinander getrennt.

Auch die stoffliche Nutzung von Methan durch aufwertende Reaktionen ist aus wirtschaftlicher Sicht von großem Interesse. So befinden sich derzeit Verfahren zur Herstellung höherer Kohlenwasserstoffe aus Methan durch oxidative Methankopplung (engl. Oxidative Coupling of Methane, OCM) in intensiver Entwicklung. Bei der oxidativen Methankopplung handelt es sich um die direkte Umsetzung von Methan in einem oxidativen, heterogen katalysierten Verfahren zu höheren Kohlenwasserstoffen. Entsprechende Verfahren erscheinen insbesondere zur Herstellung von Ethylen vielversprechend.

Bezüglich weiterer Details der oxidativen Methankopplung sei auf einschlägige Fachliteratur, beispielsweise Zavyalova, U. et al.: Statistical Analysis of Past Catalytic Data on Oxidative Methane Coupling for New Insights into the Composition of High-Performance Catalysts, ChemCatChem 3, 2011, 1935-1947, verwiesen. Auch bei der oxidativen Kopplung von Methan fallen Stoffgemische an, die zweckmäßigerweise einer thermischen Trennung unterworfen werden.

In den genannten, aber auch in einer Reihe weiterer Verfahren, bei denen der Einsatz einer thermischen Trennung zur Gewinnung einzelner Komponenten aus einem gasförmigen Stoffgemisch zweckmäßig ist, erfolgt vor der thermischen Trennung zunächst eine Verdichtung (sogenannte Rohgasverdichtung), beispielsweise auf einen Druck von 30 bis 40 bar (abs.). Vorab wird das Stoffgemisch typischerweise gekühlt und es erfolgt eine Abtrennung von schwereren Komponenten, falls erforderlich, beispielsweise von sogenanntem Pyrolyseöl. Auch nach der thermischen Trennung kann eine Verdichtung von gewonnenen Fraktionen erfolgen, die herkömmlicherweise auch als Produktverdichtung bezeichnet wird.

Bei der thermischen Trennung können unterschiedliche Kältemittel auf Kohlenwasserstoffbasis zum Einsatz kommen, beispielsweise sogenannte C3-Kältemittel (Propan und/oder Propylen) und/oder sogenannte C2-Kältemittel (Ethan und/oder Ethylen). Auch in Kältekreisläufen, in denen derartige Kältemittel bereitgestellt werden, werden Verdichter benötigt, die durch einen geeigneten Antrieb mit Leistung versorgt werden müssen. Für sämtliche Verdichtungsschritte werden herkömmlicherweise große Einwellenturboverdichter eingesetzt. Die Antriebsleistung entsprechender Verdichter liegt teilweise im hohen zweistelligen Megawattbereich, der Antrieb erfolgt in der Regel mittels Dampfturbinen.

Die Erfindung stellt sich die Aufgabe, Verfahren bzw. Anlagen zur oxidativen Kopplung von Methan effizienter durchzuführen bzw. zu betreiben.

### Offenbarung der Erfindung

Diese Aufgabe wird durch ein Verfahren und eine Anlage zur Gewinnung eines Kohlenwasserstoffprodukts gemäß den Merkmalen der unabhängigen Patentansprüche gelöst. Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Ein "Verdichter" ist eine Vorrichtung, die zum Verdichten wenigstens eines gasförmigen Stroms von wenigstens einem Eingangsdruck, bei dem dieser dem Verdichter zugeführt wird, auf wenigstens einen Enddruck, bei dem dieser dem Verdichter entnommen wird, eingerichtet ist. Ein Verdichter bildet eine bauliche Einheit, die jedoch mehrere "Verdichterstufen" in Form von Kolben-, Schrauben- und/oder Schaufelreihen (also Axial- oder Radialverdichterstufen) aufweisen kann. Insbesondere werden entsprechende Verdichterstufen mittels eines gemeinsamen Antriebs, beispielsweise über eine gemeinsame Welle, angetrieben. Ein Verdichter im erläuterten Sinn oder eine Verdichterstufe eines solchen Verdichters führt im Sprachgebrauch dieser Anmeldung einen "Verdichtungsschritt" durch.

Eine "thermische Trennung" zeichnet sich im Sprachgebrauch dieser Anmeldung dadurch aus, dass in ihr ein Gasgemisch unter zumindest teilweiser Verflüssigung aufgetrennt wird, und dass dabei ein geeignetes Kältemittel eingesetzt wird. Hierzu kommen an sich bekannte Wärmetauscher zum Einsatz. Die Trennung erfolgt mittels bekannter Trenneinheiten, beispielsweise mittels Destillationskolonnen und/oder Absorbern. In einer thermischen Trennung werden insbesondere die erwähnten C3-und/oder C2-Kältemittel eingesetzt. Diese werden zwischen unterschiedlichen Druckniveaus geführt, wobei die erwähnten Verdichter sowie beispielsweise bekannte Entspannungsturbinen oder Entspannungsventile zum Einsatz kommen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste ein. Entsprechendes gilt für Temperaturniveaus.

### Vorteile der Erfindung.

Erfindungsgemäß wurde erkannt, dass insbesondere der Einsatz einer Gasturbine zum Antreiben eines oder mehrerer der erläuterten Verdichter, die für die genannten Verdichtungsschritte zur Verdichtung eines zu trennenden Stoffgemischs, zur Verdichtung von hieraus gewonnenen Fraktionen und/oder zur Verdichtung von Kältemitteln in einem Verfahren bzw. einer Anlage zur oxidativen Kopplung von Methan eingesetzt werden, besondere und unerwartete Vorteile bieten kann. Die Grundidee der vorliegenden Erfindung besteht daher darin, einen durch eine Gasturbine angetriebenen Verdichter im Zusammenhang mit einem Verfahren bzw. in einer Anlage zur oxidativen Kopplung von Methan einzusetzen.

Die Vorteile einer Gasturbine sind hier unerwartet, da der Fachmann insbesondere aufgrund der ausgesprochen hohen Abwärmemengen, die bei der oxidativen Kopplung von Methan anfallen, eher eine Erzeugung von Dampf und die Verwendung von Dampfturbinen anstatt der erfindungsgemäß vorgeschlagenen Verwendung von Gasturbinen in Erwägung ziehen würde. Dampfturbinen wären auf den ersten Blick vorteilhaft, weil der mittels Abwärme erzeugte Dampf für den Antrieb der Dampfturbinen kostenlos zur Verfügung steht. Auf den ersten Blick erscheint es daher widersinnig, zusätzlichen Brennstoff, der auch in der oxidativen Kopplung von Methan genutzt werden könnte, für den Antrieb von Gasturbinen zu verfeuern.

Sowohl Dampfspaltverfahren als auch Verfahren zur oxidativen Kopplung von Methan umfassen ausgesprochen hohe Reaktionstemperaturen von größenordnungsmäßig 800 bis 900 °C. Ein wichtiger Unterschied besteht jedoch darin, dass die in einem Dampfspaltverfahren ablaufenden Reaktionen größtenteils endotherm, in einem Verfahren zur oxidativen Kopplung von Methan hingegen größtenteils exotherm sind. In einem Dampfspaltverfahren muss daher ständig eine große Wärmemenge mittels Brennern bereitgestellt werden, während dies in einem Verfahren zur oxidativen Kopplung von Methan nicht erforderlich ist.

Sowohl in Dampfspaltverfahren als auch in Verfahren zur oxidativen Kopplung von Methan wird kontinuierlich Verdichterleistung benötigt. Setzt man nun für den Antrieb entsprechender Verdichter Dampfturbinen ein, bestehen in einem Dampfspaltverfahren keine Schwierigkeiten, auch beim Hochfahren der Anlage sehr rasch ausreichende Dampfmengen bereitzustellen, weil hier die für die Reaktion erforderlichen Brenner vorhanden sind und ausreichende Wärmemengen liefern können. In einem Verfahren zur oxidativen Kopplung von Methan müssen hingegen zusätzliche Brenner installiert werden, die aber lediglich für das Anfahren einer entsprechenden Anlage für die Dampfproduktion benötigt werden. Nach dem Anfahren werden entsprechende Brenner nicht mehr benötigt, weil sich die Dampfmenge ausschließlich durch die Abwärme der Reaktion decken lässt und keine zusätzliche Wärmezufuhr mehr erforderlich ist. Die Brenner bleiben daher während des regulären Betriebs einer entsprechenden Anlage ungenutzt. Eine Alternative wäre, beim Anfahren auf alternative Antriebe für die Verdichter zurückzugreifen, die jedoch unter hohen Investitionskosten zusätzlich bereitgestellt werden müssten.

Außerdem wird in Dampfspaltverfahren für die durchgeführten Reaktionen Prozessdampf benötigt, der obligatorisch bereitgestellt werden muss. Es müssen daher auch aus diesem Grund geeignete Dampfsysteme bereitgestellt werden, was in einem Verfahren zur oxidativen Kopplung von Methan nicht der Fall ist.

In der Gesamtbetrachtung ist daher die Verwendung von Gasturbinen in Verfahren zur oxidativen Kopplung von Methan vorteilhaft, selbst wenn diese zusätzlichen Brennstoff erfordern, weil die Bereitstellungskosten für überwiegend nicht verwendete Brenner bzw. zusätzliche Antriebe eingespart werden.

Gasturbinen verlieren bekanntermaßen bei hoher Umgebungstemperatur erheblich an Antriebsleistung. Gasturbinen müssen daher herkömmlicherweise für die potentiell höchste Umgebungstemperatur ausgelegt werden, was jedoch zu sehr hohen Erstellungskosten einer entsprechenden Anlage führt. Zudem läuft eine derart dimensionierte Gasturbine zu Zeiten geringerer Umgebungstemperaturen tief im Teillastbereich, was zu erheblichen Wirkungsgradeinbußen führt. Herkömmlicherweise werden daher für hohe Umgebungstemperaturen keine vollen Anlagenkapazitäten garantiert, was jedoch für die erläuterten Einsatzzwecke ausgesprochen nachteilig ist, oder es wird eine Kühlung der Ansaugluft der Gasturbine eingesetzt. Die Ansaugluft wird in solchen Fällen herkömmlicherweise mit Kälteeinheiten gekühlt, welche mit Kältemitteln wie R134a oder R-290 betrieben werden.

Ein weiterer Aspekt der vorliegenden Erfindung, deren Grundidee, wie erwähnt, der Einsatz von Gasturbinen in Verfahren zur oxidativen Kopplung von Methan ist, besteht nun darin, die Kälte zur Kühlung der in der Gasturbine eingesetzten Luft Kältemittel zu verwenden, das bereits zur Kühlung in der thermischen Trennung zur Verfügung steht. Die Verdichtung eines C3-Kältemittels in einer entsprechenden thermischen Trennung erfolgt typischerweise in mehreren Schritten, insbesondere über ein Zwischendruckniveau, das beispielsweise ca. 7 bar (abs.) beträgt. Durch den Einsatz eines zusätzlichen C3-Verdampfers, beispielsweise auf diesem Zwischendruckniveau, lässt sich die für die Kühlung der Einsatzluft der Gasturbine erforderliche Kälte erzeugen. Das verdampfte Kältemittel wird anschließend erneut verdichtet bzw. in den Prozess zurückgeführt. Dient eine entsprechende Gasturbine unmittelbar einem Antrieb des Verdichters für das Kältemittel, so wird sie durch die zusätzliche zu leistende Verdichtungsarbeit zwar zusätzlich belastet, jedoch ist zu erwarten, dass die Leistungszunahme der Gasturbine durch die geringeren Ansaugtemperaturen deutlich überwiegt. Dies trifft insbesondere auf hoch effiziente Gasturbinen zu, welche aufgrund des hohen Brennkammerdrucks besonders sensitiv gegenüber Umgebungstemperaturunterschieden sind.

Durch den soeben erläuterten vorteilhaften Aspekt der vorliegenden Erfindung lässt sich insgesamt eine deutliche Verkleinerung entsprechender Antriebe erzielen, da auf die Auslegung auf die maximale Umgebungstemperatur verzichtet werden kann. Dies ermöglicht deutlich geringere Investitionskosten (CAPEX). Gleichzeitig ist stets eine maximale bzw. nahezu optimale Effizienz des Antriebs sichergestellt, da die entsprechend betriebene Gasturbine bei mittlerer Umgebungstemperatur bei Volllast anstatt bei ineffizienter Teillast betrieben werden kann. Bei mittleren Umgebungstemperaturen, die keine Kühlung erfordern, und für die eine entsprechende Gasturbine ausgelegt ist, beispielsweise im Winter, kann eine Kühlung gegebenenfalls zeitweise außer Kraft gesetzt werden, wie auch nachfolgend erläutert. Dies führt zu Einsparungen in den Betriebskosten (OPEX).

Regelungstechnisch kann sichergestellt werden, dass ein entsprechender Antrieb das gesamte Jahr über, d.h. bei unterschiedlichen Temperaturen, konstante Nennleistung liefern kann. Dies ermöglicht eine besonders einfache Handhabbarkeit des Verfahrens. Die Kühlung mittels eines auch in der thermischen Trennung eingesetzten Kältemittels hat gegenüber anderen an sich bekannten Kühlverfahren wie Verdampfungskühlung, Nebelkühlung, Sprühkühlung usw. geringeren Einfluss auf die Wartungsintervalle, insbesondere tritt kein sogenanntes Fouling auf. Eine entsprechende Anlage wird dadurch deutlich weniger wartungsintensiv.

Auch im Rahmen der vorliegenden Erfindung erfolgt vorteilhafterweise eine Erzeugung von Dampf mittels Abwärme. Da im Rahmen der vorliegenden Erfindung ein Antrieb des oder der Verdichter jedoch mittels einer Gasturbine erfolgt, kann im Normalbetrieb der Anlage der Dampf anderweitig verwendet werden, beispielsweise zur Gewinnung von Elektrizität. Die Abwärme der Gasturbine kann bei Bedarf zur Erzeugung von Dampf verwendet werden. So kann dem Mangel an Abwärme und Dampf beim Anfahren des Prozesses entgegengewirkt werden. Steht beim Anfahren also noch keine Abwärme der Reaktion zur Dampferzeugung zur Verfügung, kann stattdessen hierfür zunächst die Abwärme der Gasturbine genutzt werden, falls erforderlich.

Zur Erzielung der erläuterten Vorteile schlägt die vorliegende Erfindung ein Verfahren zur Gewinnung eines Kohlenwasserstoffprodukts vor, bei dem ein Methan enthaltendes Gasgemisch zumindest einer oxidativen Methankopplung unterworfen wird. Unter Einsatz der oxidativen Methankopplung wird ein gasförmiges Stoffgemisch erzeugt, das einem oder mehreren Verdichtungsschritten und einer thermischen Trennung unterworfen wird. In der thermischen Trennung wird, wie bereits grundsätzlich erläutert, ein Kältemittel eingesetzt, das in einem oder mehreren Verdichtungsschritten verdichtet wird. Ebenso werden in der thermischen Trennung oder anderen Trennschritten eine oder mehrere Fraktionen unter Verwendung des gasförmigen Stoffgemischs gebildet, die optional ebenfalls in einem oder mehreren Verdichtungsschritten verdichtet werden. Entsprechende Verfahren und die dabei eingesetzten Verdichter und thermischen Trennschritte wurden zuvor erläutert.

Ist hier davon die Rede, dass ein Methan enthaltendes Gasgemisch "zumindest" einer oxidativen Methankopplung unterworfen und hierdurch ein gasförmiges Stoffgemisch gebildet wird, sei darunter verstanden, dass auch zusätzliche Reaktionsschritte zur Bildung des gasförmigen Stoffgemischs eingesetzt werden können, beispielsweise einen oder mehrere sogenannte "Post Bed Cracking"-Schritte, die einer oxidativen Methankopplung nachgeschaltet sind. Zur Bildung des gasförmigen Stoffgemischs muss dabei nicht ausschließlich das Methan enthaltende Gasgemisch eingesetzt werden, beispielsweise können dem oder den "Post Bed Cracking"-Schritten zusätzliche Kohlenwasserstoffe zugeführt werden.

Erfindungsgemäß ist nun vorgesehen, dass für eine oder mehrere der genannten Verdichtungsschritte, d.h. die Verdichtungsschritte zur Verdichtung des Stoffgemischs, des Kältemittels und/oder der aus dem Stoffgemisch gebildeten Fraktion(en), falls vorhanden, ein oder mehrere Verdichter zum Einsatz kommen, die mittels einer oder mehrerer Gasturbinen angetrieben werden. Die Erfindung kann die Verwendung eines oder mehrerer entsprechender Verdichter umfassen, je nachdem, wie viele der genannten Verdichtungsschritte entsprechend erfolgen sollen. Die Vorteile der Verwendung von Gasturbinen wurden bereits erläutert.

Der oder den Gasturbinen werden, wie insoweit bekannt, atmosphärische Luft einerseits und ein Brenngas auf einem überatmosphärischen Druckniveau andererseits zugeführt. Die Luft wird gemäß einer besonders vorteilhaften Ausgestaltung der Erfindung zumindest zeitweise unter Verwendung eines Anteils des auch in der thermischen Trennung eingesetzten Kältemittels gekühlt, wie bereits ausführlich zuvor erläutert. Auch die Vorteile hiervon wurden bereits erläutert.

Besondere Vorteile lassen sich erzielen, wenn in dieser Ausgestaltung der Erfindung ein erster Anteil des Kältemittels in der thermischen Trennung und ein zweiter Anteil des Kältemittels zur Kühlung der Luft verwendet werden. Wie erläutert, kann in herkömmlichen Kältemittelkreisläufen ein Verdichter eingesetzt werden, der mehrere Verdichterstufen umfasst und damit mehrere Verdichtungsschritte implementiert. Ein derartiger Verdichter weist typischerweise mehrere Einspeisemöglichkeiten für das zuvor in einem oder mehreren Kältemittelkreisläufen verwendete Kältemittel auf. Wie grundsätzlich bekannt, kann in bekannten Anlagen beispielsweise Ethylen zur Kühlung in einem Niederdruck-, einem Mitteldruck- und einem Hochdruckkreislauf geführt werden. Ethylen aus dem Niederdruckkreislauf kann einem entsprechenden mehrstufigen Verdichter beispielsweise bei ca. 1 bar, Ethylen aus dem Mitteldruckkreislauf beispielsweise bei ca. 8 bar und Ethylen aus dem Hochdruckkreislauf beispielsweise bei ca. 23 bar, jeweils stromauf einer Verdichterstufe mit entsprechendem Saugdruck, zugeführt werden. Nach der Verdichtung auf einen Enddruck kann das als Kältemittel verwendete Ethylen auf unterschiedliche Druckniveaus gedrosselt und jeweils in den genannten Kältemittelkreisläufen verwendet werden.

Die genannten Drücke sind dabei lediglich als Beispielwerte zur Veranschaulichung einer Ausführungsform der Erfindung zu verstehen. Im Rahmen der vorliegenden Erfindung können auch andere Arten von Verdichtern und insbesondere andere Drücke verwendet werden, vor allem, wenn statt Ethylen andere Kältemittel wie Propylen zum Einsatz kommen. Die Einspeisung der Kältemittel bzw. die dabei verwendeten Einspeisedrücke richten sich nach dem Kältebedarf für die jeweiligen Kühlaufgaben. Ein entsprechender Verdichter kann auch als kombinierter Kältemittel-und Produktverdichter verwendet werden, wobei je nach dem geforderten Produktdruck zusätzliche Verdichtungsstufen vorgesehen sein können.

Gemäß der erläuterten Ausführungsform der Erfindung wird, allgemein gesprochen, vorteilhafterweise ein mehrstufiger Verdichter eingesetzt, in dem ein Kältemittel ausgehend von unterschiedlichen Ausgangsdruckniveaus auf ein Enddruckniveau verdichtet wird. Auf einem oder mehreren der Ausgangsdruckniveaus kann dabei das in der thermischen Trennung verwendete Kältemittel, auf einem oder mehreren gleichen oder unterschiedlichen Druckniveaus das für die Kühlung der Luft verwendete Kältemittel eingespeist werden. Die Einspeisung des für die Kühlung der Luft verwendeten Kältemittels kann dabei auf einem oder mehreren höheren oder niedrigeren Ausgangsdruckniveaus als die Einspeisung des in der thermischen Trennung verwendeten Kältemittels erfolgen, je nachdem, welche Temperatur für die jeweiligen Kühlaufgaben erforderlich ist und auf welchen Druck das Kältemittel daher zuvor zur Kühlung gedrosselt wurde.

Dies bedeutet, mit anderen Worten, dass für die genannten Verdichtungsaufgaben ein mehrstufiger Verdichter zum Einsatz kommen kann, wie er typischerweise in einer entsprechenden Anlage bereits vorhanden ist. Der Einsatz eines entsprechenden Verdichters ermöglicht es, auf zusätzliche Verdichtereinheiten zu verzichten und stattdessen einen bestehenden Kälteverdichter auch für die Vorkühlung der Luft für die Gasturbine einzusetzen.

Da in einer Gasturbine Brenngase verbrannt werden, könnte es unter Sicherheitsaspekten vorteilhaft sein, wenn das Kältemittel nicht direkt zur Kühlung der Luft eingesetzt wird, d.h. gemeinsam mit der Luft durch einen gemeinsamen Wärmetauscher (wenngleich durch voneinander getrennte Kanäle) geführt wird, sondern wenn Kälte von dem Kältemittel auf ein weiteres Kältemittel und anschließend auf die Luft übertragen wird. Auf diese Weise kann beispielsweise ein Kältemittel unter Sicherheitsaspekten optimiert werden. Insbesondere werden dabei im Rahmen der vorliegenden Erfindung vorteilhafterweise Wasser oder ein anderes nicht brennbares Fluid als Kältemittel verwendet.

Die vorliegende Erfindung erstreckt sich auf eine Anlage zur Gewinnung eines Kohlenwasserstoffprodukts. Eine derartige Anlage weist einen oder mehrere Reaktoren auf, die dafür eingerichtet sind, ein Methan enthaltendes Gasgemisch zumindest einer oxidativen Methankopplung zu unterwerfen, wobei unter Einsatz der oxidativen Methankopplung ein gasförmiges Stoffgemisch erzeugt wird. Das gasförmige Stoffgemisch wird einem oder mehreren Verdichtungsschritten und danach einer Tieftemperaturtrennung unterworfen. Unter Verwendung des gasförmigen Stoffgemischs werden ein oder mehrere Fraktionen gebildet und optional ebenfalls verdichtet. Eine entsprechende Anlage ist dafür eingerichtet, in der thermischen Trennung ein Kältemittel einzusetzen, das in einem oder mehreren Verdichtungsschritten verdichtet wird.

Erfindungsgemäß sind zur Durchführung eines oder mehrerer der Verdichtungsschritte ein oder mehrere Verdichter bereitgestellt und mit einer oder mehreren Gasturbinen gekoppelt, die zum Antreiben des oder der Verdichter eingerichtet sind. Auch hier gilt, dass, je nach Anzahl der Verdichtungsschritte, ein oder mehrere entsprechender Verdichter bereitgestellt sein können. Ferner sind Mittel vorgesehen, die dafür eingerichtet sind, der oder den Gasturbinen atmosphärische Luft und ein Brenngas auf einem überatmosphärischen Druckniveau zuzuführen.

Gemäß einer Ausführungsform der Erfindung ist eine Kühleinheit ist bereitgestellt, die dafür eingerichtet ist, die Luft zumindest zeitweise unter Verwendung eines Anteils des auch in der thermischen Trennung eingesetzten Kältemittels zu kühlen.

Eine derartige Anlage ist gemäß einer besonders bevorzugten Ausführungsform zur Durchführung eines Verfahrens eingerichtet, wie es zuvor erläutert wurde. Eine entsprechende Anlage profitiert insgesamt von den zuvor erläuterten Vorteilen, auf die daher ausdrücklich verwiesen wird.

Die Erfindung wird nachfolgend unter Bezugnahme auf die beigefügte Zeichnung näher erläutert, in der bevorzugte Ausführungsformen der Erfindung schematisch veranschaulicht sind.

### Kurze Beschreibung der Zeichnungen

In Figur 1 ist ein Verfahren einer Ausführungsform der Erfindung in Form eines schematischen Ablaufplans veranschaulicht.
In Figur 2 ist ein Verfahren einer Ausführungsform der Erfindung in Form eines schematischen Ablaufplans veranschaulicht.

### Ausführliche Beschreibung der Zeichnungen

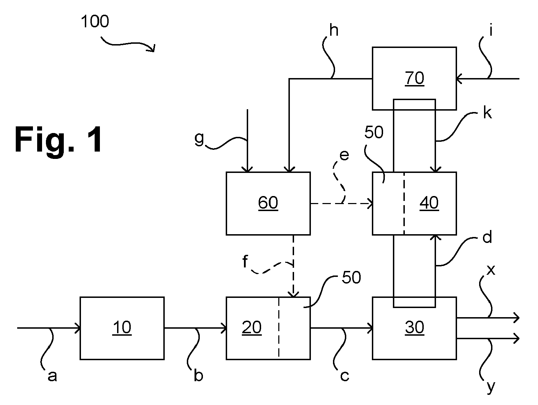

In Figur 1 ist Verfahren gemäß einer Ausführungsform der Erfindung in Form eines schematischen Ablaufplans veranschaulicht und insgesamt mit 100 bezeichnet.

In dem Verfahren 100 wird ein Verfahren 10 zur oxidativen Kopplung von Methan durchgeführt. Dem Verfahren 10 zur oxidativen Kopplung von Methan wird ein Methan enthaltendes Gasgemisch in Form eines Fluidstroms, der hier mit a bezeichnet ist, zugeführt. Unter Einsatz des Verfahrens 10 zur oxidativen Kopplung von Methan, dem auch weitere Ströme zugeführt werden können, beispielsweise Recycleströme und/oder Sauerstoffströme, wird ein gasförmiges Stoffgemisch erzeugt, das in Form eines Stroms b bereitgestellt werden kann. Das Stoffgemisch des Stroms b kann einem oder mehreren nicht dargestellten Aufbereitungsschritten unterworfen werden. Das Stoffgemisch des Stroms b wird anschließend einem oder mehreren Verdichtungsschritten 20 unterworfen und hier auf einen Druck von beispielsweise ca. 30 bis 40 bar (abs.) verdichtet. Anschließend wird das Stoffgemisch, nun in Form eines Stroms c veranschaulicht, einer thermischen Trennung 30 zugeführt.

Die thermische Trennung 30 erfolgt unter Verwendung eines oder mehrere Kältemittel, das oder die in Form eines Kreislaufs verwendet werden, von dem hier ein Strom d schematisch veranschaulicht ist. Entspannungsschritte zur Entspannung des Kältemittels des Stroms d sind nicht dargestellt, das Kältemittel des Stroms d wird jedoch ebenfalls einem oder mehreren Verdichtungsschritten 40 unterworfen.

Gemäß der hier veranschaulichten besonders bevorzugten Ausführungsform der Erfindung ist eine Gasturbine 60 vorgesehen, welche mechanische Leistung, hier in Form der gestrichelten Pfeile e und f veranschaulicht, an einen in dem oder den Verdichtungsschritten 20 und/oder dem oder den Verdichtungsschritten 40 verwendeten Verdichter 50 bereitstellen kann. Der Gasturbine 60 werden dabei ein Brenngasstrom, hier mit g veranschaulicht, und ein Strom atmosphärischer Luft, hier mit h veranschaulicht, zugeführt. Eine Verdichtung des Brenngasstroms g und des Luftstroms h ist nicht veranschaulicht, kann jedoch ebenfalls vorgesehen sein.

Ein wesentlicher Aspekt der in Figur 1 veranschaulichten Ausführungsform der Erfindung ist eine Vorkühlung des Luftstroms h in einem oder mehreren Vorkühlschritten 70. Dem oder den Vorkühlschritten 70 wird ein Einsatzluftstrom i, beispielsweise auf Umgebungstemperatur oder oberhalb der Umgebungstemperatur, zugeführt, wie erläutert. In dem Kühlschritt 70 kommt ein Kältemittel zum Einsatz, das in Form eines Stroms k veranschaulicht ist und ebenfalls aus dem oder den Verdichtungsschritten 40 stammen kann. Mit anderen Worten wird in der dargestellten Ausführungsform in der Tieftemperaturtrennung 30 und dem Kühlschritt 70 dasselbe Kältemittel eingesetzt, das jedoch in unterschiedlichen Anteilen und ggf. auf unterschiedlichen Druckniveaus bereitgestellt wird.

In der Tieftemperaturtrennung 30 werden in oder mehrere Fraktionen aus dem in Form des Stroms c bereitgestellten Gasgemisch gebildet, die in Form der Ströme x und y veranschaulicht sind. Auch diese können verdichtet werden, wie erläutert.

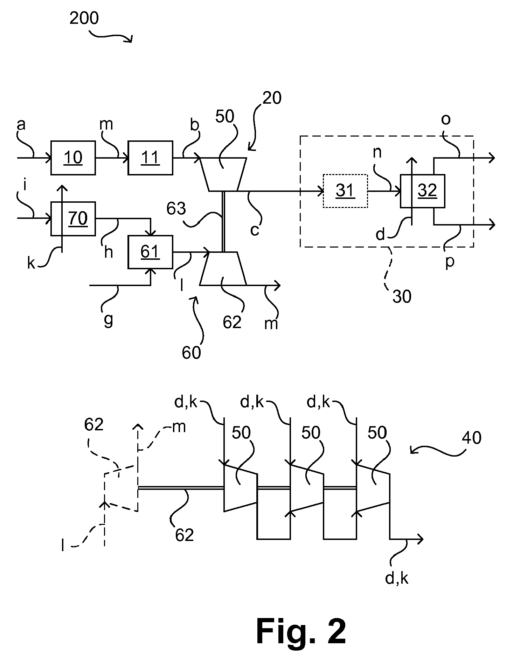

In Figur 2 ist ein Verfahren gemäß einer Ausführungsform der Erfindung in alternativer Darstellung veranschaulicht. Das Verfahren ist insgesamt mit 200 bezeichnet. In Figur 2 sind insbesondere die Verdichtungsschritte 20 und 40 mit den jeweils eingesetzten Apparaten detaillierter dargestellt.

Wie veranschaulicht, sind in den Verdichtungsschritten 20 und 40 die Verdichter 50 bzw. entsprechende Verdichterstufen 50 mit einer Entspannungsstufe 62 der Gasturbine 60 jeweils über eine Welle 63 gekoppelt. Wie in Figur 2 bezüglich des Verdichtungsschritts 40 veranschaulicht, können die Ströme d und k, also der für die thermische Trennung eingesetzte Anteil und der für die Kühlung der Luft eingesetzte Anteil des Kältemittels, stromauf unterschiedlicher Verdichterstufen dem Verdichtungsschritt 40 zugeführt werden. Je nach Bedarf und dem erforderlichen Kältebedarf sind beliebige Varianten denkbar. Stromauf jeder Verdichterstufe können jeweils einer der Ströme d und k oder beide gemeinsam eingespeist werden. Es kann sich auch jeweils nur um Anteile der Ströme d bzw. k handeln.

Wie in Figur 2 bezüglich der Gasturbine 60 veranschaulicht, werden hier die Ströme h und g einer Brennkammer 61 zugeführt. Ein hier erhaltener Abgasstrom I wird in der Entspannungsstufe 62 der Gasturbine 60 zu einem Strom m entspannt.

Ferner ist in der Figur 2 eine Ausführungsform der Tieftemperaturtrennung 30 detaillierter dargestellt. Das unter Einsatz des Verfahrens 10 zur oxidativen Methankopplung gebildete Gasgemisch des Stroms c umfasst unter anderem Methan und höhere Kohlenwasserstoffe, insbesondere die Zielverbindung Ethylen. Dieses Gasgemisch kann in der thermischen Trennung 30 beispielsweise zunächst einem oder mehreren optionalen Vortrennschritten 31 zugeführt werden.

Ein auf diese Weise gebildeter Strom n kann einem Trennschritt 32 zugeführt werden, in dem ein überwiegend Methan und Wasserstoff enthaltender Strom o (der insgesamt oder teilweise zur Bildung des in das Verfahren 10 zur oxidativen Methankopplung zurückgeführt werden kann) und ein überwiegend oder ausschließlich Kohlenwasserstoffe mit zwei Kohlenstoffatomen enthaltender Strom p gebildet werden. Aus dem Strom p kann insbesondere die Zielverbindung Ethylen abgetrennt werden.

Zumindest der Strom p kann in einem oder mehreren Verdichtungsschritten verdichtet werden, die analog wie die Verdichtungsschritte 20 und 30 durchgeführt werden können.

## Patentansprüche

1. Verfahren (100, 200) zur Gewinnung eines Kohlenwasserstoffprodukts, bei dem ein Methan enthaltendes Gasgemisch zumindest einer oxidativen Methankopplung (10) unterworfen wird, und bei dem unter Einsatz der oxidativen Methankopplung (10) ein gasförmiges Stoffgemisch erzeugt wird, das einem oder mehreren Verdichtungsschritten (20) und einer thermischen Trennung (30) unterworfen wird, wobei in der thermischen Trennung (30) ein Kältemittel eingesetzt wird, das in einem oder mehreren Verdichtungsschritten (40) verdichtet wird, und wobei unter Verwendung des Stoffgemischs eine oder mehrere Fraktionen gebildet werden, die optional in einem oder mehreren weiteren Verdichtungsschritten verdichtet werden, **dadurch gekennzeichnet, dass** für einen oder mehrere der Verdichtungsschritte (20, 30) ein oder mehrere Verdichter (50) zum Einsatz kommen, die mittels einer oder mehrerer Gasturbinen (60) angetrieben werden.

2. Verfahren (100, 200) nach Anspruch 1, bei dem der oder den Gasturbinen (60) atmosphärische Luft und ein Brenngas zugeführt werden und die Luft zumindest zeitweise unter Verwendung eines Anteils des auch in der thermischen Trennung (30) eingesetzten Kältemittels gekühlt wird.

3. Verfahren (100, 200) nach Anspruch 1 oder 2, bei dem ein erster Anteil des Kältemittels in der thermischen Trennung (30) und ein zweiter Anteil des Kältemittels zur Kühlung der Luft verwendet wird, wobei der erste Anteil des Kältemittels und der zweiter Anteil des Kältemittels in dem oder den Verdichtungsschritten (40) jeweils von einem oder mehreren Ausgangsdruckniveaus auf ein Enddruckniveau verdichtet werden.

4. Verfahren (100, 200) nach Anspruch 3, bei dem für die Verdichtungsschritte (40) ein gemeinsamer Verdichter eingesetzt wird.

5. Verfahren (100, 200) nach einem der Ansprüche 2 bis 4, bei dem eine Menge des Kältemittels zur Kühlung der Luft in Abhängigkeit von einer Differenztemperatur zwischen einem Zieltemperaturniveau und der Temperatur eines Luftvolumens, der die Luft entnommen wird, eingestellt wird.

6. Verfahren (100, 200) nach einem der vorstehenden Ansprüche, bei dem als Kältemittel ein Propan und/oder Propylen enthaltendes Fluid eingesetzt wird.

7. Verfahren (100, 200) nach einem der Ansprüche 2 bis 6, bei dem die Luft unter Verwendung des Kältemittels gekühlt wird, indem Kälte von diesem auf ein weiteres Kältemittel und anschließend auf die Luft übertragen wird.

8. Verfahren (100, 200) nach Anspruch 7, bei dem als weiteres Kältemittel Wasser oder ein anderes nicht brennbares Fluid verwendet wird.

9. Anlage zur Gewinnung eines Kohlenwasserstoffprodukts, mit einem oder mehreren Reaktoren, die dafür eingerichtet sind, ein Methan enthaltendes Gasgemisch einer oxidativen Methankopplung (10) zu unterwerfen und unter Einsatz der oxidativen Methankopplung (10) ein gasförmiges Stoffgemisch zu erzeugen, und mit Mitteln, die dafür eingerichtet sind, das Stoffgemisch einem oder mehreren Verdichtungsschritten (20) und einer thermischen Trennung (30) zu unterwerfen, wobei eine Kältemitteleinheit vorgesehen ist, die dafür eingerichtet ist, in der thermischen Trennung (30) ein Kältemittel einzusetzen, das in einem oder mehreren Verdichtungsschritten (40) verdichtet wird, und wobei Mittel vorgesehen sind, die dafür eingerichtet sind, unter Verwendung des Stoffgemischs eine oder mehrere Fraktionen zu bilden und diese optional in einem oder mehreren weiteren Verdichtungsschritten zu verdichten, **dadurch gekennzeichnet, dass** zur Durchführung eines oder mehrerer der Verdichtungsschritte (40) ein oder mehrere Verdichter (50) bereitgestellt sind, die mit einer oder mehreren Gasturbinen (60) gekoppelt sind, die zum Antrieb des oder der Verdichter eingerichtet sind.

10. Anlage nach Anspruch 9, die dafür eingerichtet ist, der oder den Gasturbinen (60) atmosphärische Luft und ein Brenngas auf einem überatmosphärischen Druckniveau zuzuführen und die Luft zumindest zeitweise unter Verwendung eines Anteils des auch in der thermischen Trennung (30) eingesetzten Kältemittels zu kühlen.

11. Anlage nach Anspruch 10, die zur Durchführung eines Verfahrens nach einem der Ansprüche 1 bis 9 eingerichtet ist.
